Europäisches Patentamt

European Patent Office

Office européen des brevets

(19)

(11) Veröffentlichungsnummer: **0 338 307**
**A1**

# (12) EUROPÄISCHE PATENTANMELDUNG

(21) Anmeldenummer: **89105796.0**

(22) Anmeldetag: **03.04.89**

(51) Int. Cl.4: **C12P 19/04 , C12P 19/06**

(30) Priorität: **16.04.88 DE 3812682**

(43) Veröffentlichungstag der Anmeldung:
**25.10.89 Patentblatt 89/43**

(84) Benannte Vertragsstaaten:
**AT DE FR GB NL**

(71) Anmelder: **Wolff Walsrode Aktiengesellschaft**
**Postfach**
**D-3030 Walsrode 1(DE)**

(72) Erfinder: **Wilke, Michaela, Dr.**
**Ahornweg 9**
**D-3034 Schneverdingen(DE)**
Erfinder: **Szablikowski, Klaus, Dr.**
**Claudiusstrasse 5**
**D-3030 Walsrode 1(DE)**
Erfinder: **Balser, Klaus, Dr.**
**Geibelstrasse 24**
**D-3030 Walsrode 1(DE)**

(74) Vertreter: **Zobel, Manfred, Dr. et al**
**c/o BAYER AG Konzernverwaltung RP**
**Patentabteilung Bayerwerk**
**D-5090 Leverkusen(DE)**

(54) **Verfahren zur Herstellung von Heteropolysacchariden mit verbesserten Eigenschaften, insbesondere Xanthan.**

(57) Cellulasefreie Heteropolysaccharide, insbesondere Xanthan, sind erhältlich durch Behandlung bei speziellen pH-Werten, Temperaturbehandlung, gegebenenfalls Abkühlung, Neutralisation udn gegebenenfalls anschließende Fällung in einem hochturbulenten Fällbad.

EP 0 338 307 A1

# Verfahren zur Herstellung von Heteropolysacchariden mit verbesserten Eigenschaften, insbesondere Xanthan

Die Erfindung betrifft ein Verfahren zur Herstellung von Heteropolysacchariden, insbesondere Xanthan und ein cellulasefreies Xanthan mit verbesserten Eigenschaften.

Heteropolysaccharide, die durch Fermentation von Mikroorganismen erhältlich sind, sind wegen ihrer günstigen Eigenschaften für zahlreiche Anwendungen geeignet. Bekannte Heteropolysaccharide sind beispielsweise Dextran, Pullulan, Curdlan, Scleroglucan, insbesondere Xanthan.

Xanthan ist ein exocelluläres Heteropolysaccharid, das durch Fermentation von Xanthomonas campestris oder anderen Xanthomonas-Stämmen erhältlich ist. Xanthan wird in industriellem Maßstab produziert und z.B. zur Viskositätseinstellung in Lebensmitteln, aber auch in der Erdölförderung eingesetzt.

Xanthan, seine Herstellung und Verwendung wird beispielsweise beschrieben in GB-A-2 052 542, GB-PS 1 487 530, DE-A-2 737 989 und Biotechnology Vol. 2, 1985, Verlag Chemie, Weinheim, S. 616 f. Die Fermentation eines z.B. Xanthan produzierenden Mikroorganismus ergibt im allgemeinen eine viskose Kulturlösung, in der außer dem Heteropolysaccharid Begleitstoffe, wie z.B. nicht verbrauchte Komponenten der Nährlösung oder Zellen bzw. Zellreste oder auch Fermentationsnebenprodukte enthalten sein können.

Eine übliche Methode für die Reinigung von Heteropolysacchariden, insbesondere Xanthan wird z.B. beschrieben in Biotechnology, Vol. 3, herausgegeben von H.J. Rehm und G. Reed, 1983, Verlag Chemie Weinheim; S. 548 f. Diese Methode besteht darin, das Heteropolysaccharid durch Zugabe von Isopropanol auszufällen, abzufiltrieren, überschüssiges Fällmittel abzutrennen und den verbleibenden Rückstand zu einem trockenen Produkt aufzuarbeiten. Diese Produktaufarbeitung ergibt ein Produkt, das im wiedergelösten Zustand eine hellgelbe, trübe Lösung liefert. Ferner ist die Filtrierbarkeit durch feine Filter stark behindert. Somit ist es vorteilhaft, wenn das isolierte trockene Xanthan bei seiner Auflösung in Wasser möglichst wenige Gelteilchen, auch Mikrogel oder micellenartige Teilchen genannt, enthält. Das Vorhandensein dieser Gelteilchen beeinflußt die Filtrierbarkeit verdünnter wäßriger Xanthan-Lösung in erheblichem Maß und kann im Extremfall zum Verblocken des Filters führen. Auch hat das Produkt den Nachteil, mit Cellulasen behaftet zu sein, die während der Kultivierung der Xanthomonas-Bakterien als Nebenprodukte anfallen. Während der bekannten Isolierungsverfahren werden diese Enzyme nicht abgetrennt. Für den Anwendungsfall allerdings ist die Gegenwart dieser celluloseabbauenden Enzymsysteme nachteilig.

Insbesondere dann ist das Vorhandensein von Cellulasen negativ zu bewerten, wenn eine Lösung von Xanthan in Kombination mit einem $\beta$-glykosidisch verknüpften Polysaccharid, z.B. Cellulose oder einem Cellulosederivat, gebracht werden soll. Innerhalb kürzerer Zeit bewirken die in der Xanthan-Lösung vorhandenen Cellulasen einen Abbau der Cellulose bzw. des Cellulose-Derivates.

Es ist bekannt, die in einer Xanthan-Lösung vorhandenen Cellulasen durch den Einsatz von Alkalihypochlorit, wie in GB-A-2 052 542 beschrieben, zu inhibieren. Auch ist in GB-PS 1 487 530 die Behandlung von festem Xanthan mit z.B. Propylenoxid beschrieben.

Nachteilig an diesen Methoden ist entweder die mangelnde Effizienz oder aber der nur noch bedingt gegebene lebensmittelrechtliche Status des Endproduktes. Damit wird die Anwendungsbreite eines so vorbehandelten Produktes eingeschränkt.

Es wird zwar in DE-A-2 737 989 eine Filtration vor der eigentlichen Fällung beschrieben. Die dort beschriebene Methode arbeitet mit Filtermitteln auf Basis Kieselgur.

Nachteilig an dem erwähnten Verfahren ist allerdings, daß bei relativ hohen Temperaturen von mindestens 112°C während des Filtrationsvorganges gearbeitet werden soll. Bei diesen hohen Temperaturen ist ein Abbau des Xanthan-Moleküls zu erwarten.

Wie oben angegeben, ist es beim dem Standardverfahren üblich, die Kulturlösung in ein geeignetes Fällmittel unter Rühren einzutragen und die dabei entstehende mehr oder weniger grob faserige und gelbgefärbte Xanthan-Fällung anschließend abzutrennen und zu trocknen.

Nachteilig an diesem Verfahren ist zum einen das Entstehen eines grobfaserigen Produktes mit der Gefahr, daß im Innern der Faser noch nicht ausgefälltes, gelartiges Xanthan vorliegt, da der Fällvorgang noch nicht abgeschlossen war. Zum anderen wird bei diesem Verfahren relativ viel von dem bei der Fermentation mit Xanthomonas campestris-Kulturen als Nebenprodukt entstehenden gelben Farbstoff in die Fasern eingelagert.

Der Erfindung lag die Aufgabe zugrunde, ein verbessertes Verfahren zur Aufarbeitung von Heteropolysacchariden, insbesondere Xanthan bereitzustellen und ein verbessertes Xanthan.

Gegenstand der Erfindung ist ein Verfahren zur Herstellung eines cellulasefreien Heteropolysaccharides welches durch Fermentation von Mikroorganismen erhältlich ist, dadurch gekennzeichnet ist, daß man

a) eine das Heteropolysaccharid enthaltene Lösung auf einen pH-Wert außerhalb des Bereiches von 5 bis 9, einstellt

b) anschließend eine Temperaturbehandlung bei erhöhter Temperatur durchführt

c) gegebenenfalls abkühlt

d) gegebenenfalls neutralisiert und gegebenenfalls

e) das so erhaltene Produkt in ein hochturbulentes Fällbad zur Ausfällung des Heteropolysaccharids einträgt.

Insbesondere wenn ein klarlösliches Produkt erhalten werden soll, wird in einem Schritt f) vorzugsweise vor dem Schritt a) oder nach dem Schritt d) die das Heteropolysaccarid enthaltende Lösung einer Filtation unterworfen.

Ein besonders bevorzugtes Heteropolysaccharid ist Xanthan.

In einer besonders bevorzugten Ausführungsform wird das Heteropolysaccharid, insbesondere Xanthan in Stufe a) mit einer Säure auf einen pH-Wert von 1 bis 3, insbesondere 1 bis 2 eingestellt. Bevorzugte Säuren sind anorganische Säuren wie Salzsäure und Salpetersäure sowie organische Säuren wie Brenztraubensäure und Essigsäure.

In einer besonders bevorzugten Ausführungsform wird in Stufe b) das Produkt bei einer Temperatur von 40 bis 120 °C, insbesondere 60 bis 100 °C, erhitzt, vorzugsweise 10 bis 120 Minuten, insbesondere 45 bis 90 Minuten.

Das Fällbad in Stufe e) besteht vorzugsweise aus einem Alkohol, insbesondere Isopropanol.

Geeignete Filter für Schritt f) sind insbesondere Membranfilter. Die Porenweite dieser Filter liegt vorzugsweise zwischen 0,1 und 1,2 μm, insbesondere zwischen 0,3 und 0,8 μm. Bevorzugt ist hierbei die beispielsweise aus Bioingeneering vom Heft 1 Jahrgang 1987 S. 62 bekannte Cross-flow-Filtrationsmethode, obwohl grundsätzlich auch die statische Filtrationsmethode möglich ist.

Die Konzentration der Kulturlösung an Xanthan sollte nicht zu hoch liegen, da die mit höheren Xanthan-Konzentrationen verbundene höhere Viskosität der Lösung beim Filtrieren nachteilig ist. Bevorzugt sind Xanthan-Konzentrationen von 0,1 bis 3 %, insbesondere von 0,5 bis 1,5 %.

Durch die Filtration f) wird ein Teil der in einer Kulturlösung vorhandenen störenden Begleitstoffe, z.B. Zellreste, abgetrennt, und man erhält ein im Vergleich zur Kulturlösung deutlich klareres Filtrat.

Zur Inaktivierung der gegebenenfalls vorhandenen Enzyme, insbesondere Cellulasen, wird das Produkt auf einen pH-Wert außerhalb des obengenannten Bereiches eingestellt, anschließend erhitzt und dann vorzugsweise abgekühlt und gegebenenfalls mit einer geeigneten Base neutralisiert.

Erfindungsgemäß kann die Ausfällung des Heteropolysaccharids, insbesondere Xanthan, aus der aufbereiteten Kulturlösung erfolgen, indem das Fällmedium durch geeignete Aggregate in Turbulenz versetzt wird und dann die Kulturlösung in dosiertem Zulauf eingetragen wird.

Als Fällmittel eignen sich die bekannten Fällmittel, bevorzugt Isopropanol, Methanol und Aceton.

Das Eintragen der Kulturlösung erfolgt vorteilhafterweise durch Druck über ein geeignetes Düsensystem.

Das Fällmittel wird z.B. durch Einsatz geeigneter Mischwerke, die gleichzeitig eine nur geringe Scherbelastung des zu mischenden Gutes bedingen, in große Turbulenzen versetzt. Besonders geeignete Vorrichtungen zur Erzeugung der erforderlichen hohen Turbulenz sind Leitstrahlmischwerke.

Die erforderliche hohe Turbulenz ist dadurch gekennzeichnet, daß das gesamte Fällmedium stark verwirbelt wird und durch völlig unregelmäßige Bewegungsformen charakterisiert ist. Benachbarte Strömungsgebiete werden schnell miteinander vermischt.

Gegenstand der Erfindung ist weiterhin ein klar lösliches cellulasefreies Xanthan, welches nach dem erfindungsgemäßen Verfahren erhältlich ist und

1. in 1 %iger wäßriger Lösung eine Extinktion von maximal 0,17 gemessen bei 600 nm (Schichtdicke 1,0 cm mit einem Spektralphotometer) aufweist und

2. keine aktive Cellulasenaktivität aufweist.

Das erfindungsgemäße Xanthan ist feinfaserig und weiß gefärbt. Im Unterschied zu den erwähnten Standardausfällverfahren von Xanthan bedingt das erfindungsgemäße Fällverfahren eine wesentlich kürzere Ausfällzeit.

Das nach der Durchführung des erfindungsgemäßen Verfahrens erhaltene ausgefällte Produkt, insbesondere Xanthan, kann aus dem Fällmedium durch übliche Trennverfahren z.B. Filtration abgetrennt und nach üblichen bekannten Verfahren getrocknet und konfektioniert werden.

Die erfindungsgemäß erhältlichen Heteropolysaccharide, insbesondere das erfindungsgemäße Xanthan, zeichnen sich beim Lösen in Wasser durch eine ausgezeichnete Klarlöslichkeit und gute Filtrierbarkeit aus. Weiterhin ist aufgrund der Inaktivierung der Cellulasen eine viskositätsstabile Kombination mit Cellulose bzw. Cellulosederivaten, z.B. Carboxymethylcellulose vorhanden.

Die durch diese Behandlungsmethode erreichte irreversible Inaktivierung der Cellulasen ist daran nachweisbar, daß man an einer Lösung aus den erfindungsgemäß erhältlichen Heteropolysacchariden, insbesondere Xanthan, und Cellulose bzw.

Cellulosederivaten nach Lagerung keine Viskositätsänderung feststellt. Im Rahmen der vorliegenden Erfindung wird von einer vollständigen Inaktivierung von Cellulasen ausgegangen, wenn eine Abmischung der erfindungsgemäß erhaltenen Heteropolysaccharide und von Cellulose bzw. Carboxymethylcellulose zu gleichen Teilen nach einer 40 stündigen Lagerung bei 20°C eine Viskositätsänderung gemessen nach einer üblichen Methode zur Viskositätsbestimmung z.B. mit einem Rotationsviskosimeter von maximal 5 % aufweist.

Beispiel 1

1 kg einer wäßrigen Kulturlösung von Xanthomonas campestris mit einer Konzentration von 2,0 % an Xanthan wurde mit 1 kg destilliertem Wasser verdünnt. Diese Lösung wurde durch eine Membranfilter mit einer Porenweite von 0,45 μm nach dem Verfahren der Querstrom-Filtration filtriert. Das dabei gewonnene klare Filtrat wurde mit Salpetersäure versetzt und auf pH 1,5 eingestellt. Anschließend wurde auf 85°C für 70 Minuten erhitzt. Nach Abkühlen auf Raumtemperatur erfolgte die Neutralisation mit Natronlauge.

Für die sich anschließende Fällung wurde 90 %iges Isopropanol mit Hilfe eines Mischwerkes (Firma Ystral Typ Leitstrahlmischer) in Turbulenz versetzt. In dieses Fällbad wurde die vorbehandelte Kulturlösung über ein geeignetes Düsensystem eingetragen.

Bei dieser Fällmethode fällt das Xanthan in Form feiner weißer Fasern an. Nach Beendigung des Eindüsens der Kulturlösung ist der Fällvorgang beendet. Das ausgefallene Xanthan wird durch eine Filtration abgetrennt, anschließend in einem Trockenofen bei 50°C getrocknet und abschließend einer Zerkleinerung unterworfen.

Das gewonnene fast weiße, pulverförmige Xanthan löst sich in Wasser zu einer hochviskosen, klaren Lösung. Nach Vermischen mit Carboxymethylcellulose zu gleichen Teilen in Lösung tritt bis zu 40 Stunden eine Viskosi tätserniedrigung von maximal 5 % auf. Die Extinktion beträgt 0,13. Die Filtrierbarkeit einer 0,25 %igen Lösung durch ein 1,2 μm-Membranfilter bei 1 bar Überdruck ergab einen Filterwert von 7 g.

Bei diesem Filtrationstest werden die reziproken Werte der Filtratmenge gegen die reziproken Zeitwerte in einem Diagramm aufgetragen. Der Filterwert gibt den Ordinatenabschnitt (1/t = 0) an.

Beispiel 2 (Vergleichsbeispiel)

1 kg einer Kulturlösung von Xanthomonas campestris wurde unter Rühren in 90 %iges Isopropanol eingetragen. Zur Vervollständigung des Fällvorganges wird noch 1 Stunde nachgerührt. Anschließend wird das ausgefallene Xanthan durch Filtration vom Fällmedium abgetrennt.

Bei dieser Fällmethode fällt das isolierte Xanthan in Form grober gelbgefärbter Fasern an. Eine aus diesem trockenen, pulverförmigen Xanthan hergestellte Lösung ist trübe und gelbgefärbt.

Eine Abmischung dieser Xanthan-Lösung mit Carboxymethylcellulose-Lösung ergibt innerhalb von 40 Stunden einen deutlichen Viskositätsabfall um ca. 32 % des Ausgangswertes.

Als Filterwert wurden 3.31 g Polymer erhalten. Die Extinktion beträgt 1,40.

## Ansprüche

1. Verfahren zur Herstellung von cellulasefreien Heteropolysacchariden, dadurch gekennzeichnet, daß man

a) eine das Heteropolysaccharid enthaltene Lösung auf einen pH-Wert außerhalb eines Bereiches von 5 bis 9, einstellt

b) anschließend eine Temperaturbehandlung bei erhöhter Temperatur durchführt

c) gegebenenfalls abkühlt

d) gegebenenfalls neutralisiert und gegebenenfalls

e) das Produkt in ein turbulentes Fällbad einträgt.

2. Verfahren nach Anspruch 1, dadurch gekennzeichnet, daß man in einem Schritt f) der vor dem Schritt a) oder nach dem Schritt d) liegt, die das Heteropolysaccharid enthaltende Lösung einer Filtration unterwirft.

3. Verfahren nach wenigstens einem der vorhergehenden Ansprüche, dadurch gekennzeichnet, daß das Heteropolysaccharid Xanthan ist.

4. Verfahren nach wenigstens einem der vorhergehenden Ansprüche, dadurch gekennzeichnet, daß das Heteropolysaccharid in Stufe a) mit einer Säure auf einen pH-Bereich von 1 bis 2 eingestellt wird.

5. Verfahren nach wenigstens einem der vorhergehenden Ansprüche, dadurch gekennzeichnet, daß die Temperatur in Stufe b) 10 bis 120 Minuten auf 40 bis 120°C eingestellt wird.

6. Verfahren nach wenigstens einem der vorhergehenden Ansprüche, dadurch gekennzeichnet, daß das Fällbad in Stufe e) im wesentlichen aus Alkohol, insbesondere Isopropanol besteht.

7. Verfahren nach wenigstens einem der vorhergehenden Ansprüche, dadurch gekennzeichnet, daß das Fällbad mit Hilfe eines Mischwerkes in Turbulenz versetzt wird.

8. Klarlösliches cellulasefreies Xanthan, welches in 1 %iger wäßriger Lösung eine Extinktion von maximal 0,17 gemessen bei 600 nm (Schichtdicke 1 cm) aufweist und keine aktiven Cellulasen aufweist.

9. Verwendung des Xanthans gemäß Anspruch 8 als Zusatz für Cellulose- bzw. Cellulosederivathaltige Produkte.

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| X | US-A-3 964 972 (J.T. PATTON) <br> * Tabelle 3; Ansprüche * <br> --- | 1,3,5,6 ,8 | C 12 P 19/04 <br> C 12 P 19/06 |
| X | EP-A-0 215 692 (RHONE-POULENC) <br> * Ansprüche * <br> --- | 1,3-6,8 | |
| X | EP-A-0 140 724 (RHONE-POULENC) <br> * Ansprüche; Seite 3; Seite 6, Zeilen 14-18 * <br> --- | 1-3,5,6 ,8 | |
| X | EP-A-0 048 616 (MERCK) <br> * Anspruch 1; Seite 2, Zeilen 23-25 * <br> ------ | 1,3,5,8 ,9 | |

RECHERCHIERTE SACHGEBIETE (Int. Cl.4)

C 12 P
C 08 B

Der vorliegende Recherchenbericht wurde für alle Patentansprüche erstellt

| Recherchenort | Abschlußdatum der Recherche | Prüfer |
|---|---|---|
| DEN HAAG | 04-07-1989 | SOMERVILLE F.M. |